# EUROPÄISCHE PATENTSCHRIFT

(11) **EP 2 895 117 B1**
(45) Veröffentlichungstag und Bekanntmachung des Hinweises auf die Patenterteilung: **05.10.2016**
(21) Anmeldenummer: 13776491.6
(22) Anmeldetag: 14.10.2013
(51) Int. Cl.: A61F 2/90, A61F 2/91, A61F 2/915, A61F 2/844

(54) **STENT**
STENT
STENT

(30) Priorität: 05.11.2012 DE 102012220129
(43) Veröffentlichungstag der Anmeldung: 22.07.2015
(73) Patentinhaber: Variomed AG, 9496 Balzers (LI)
(72) Erfinder: FISCHER, Harald, 76356 Weingarten (DE)
(74) Vertreter: Manitz, Finsterwald & Partner GbR
(86) Internationale Anmeldenummer: PCT/EP2013/071407
(87) Internationale Veröffentlichungsnummer: WO 2014/067770

(56) Entgegenhaltungen:
- EP-A1- 0 832 618
- US-A1- 2002 123 792

## Beschreibung

Die vorliegende Erfindung betrifft einen Stent zur transluminalen Implantation in Hohlorgane, insbesondere in Venen oder allgemein in Blutgefäße, Harnleiter, Speiseröhren, Colon, Duodenum oder Gallenwege, mit einem im Wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist. Stents dieser Art werden zur Rekanalisation von krankhaft veränderten Hohlorganen eingesetzt. Dabei werden die Stents im komprimierten Zustand über einen Einführkatheter an die zu behandelnde Stelle innerhalb des Hohlorgans eingeführt, wo sie durch unterschiedliche Maßnahmen auf einen Durchmesser, der dem Durchmesser des gesunden Hohlorgans entspricht, expandiert werden, so dass eine Stützwirkung des Hohlorgans, beispielsweise einer Gefäßwand, erreicht wird. Derartige Stents können z.B. dadurch erzeugt werden, dass in die Wand eines röhrenförmigen Körpers Durchbrechungen wie z.B. Schlitze geschnitten werden, die sich teilweise in Längsrichtung des Stents erstrecken, so dass bei der Expansion des Stents beispielsweise rautenförmige Durchbrechungen entstehen.

Um diese Stützwirkung gewährleisten zu können, müssen die Stents in der Lage sein, eine ausreichende radiale Aufstellkraft auszuüben, die einer von der Gefäßwand ausgeübten radialen Krafteinwirkung entgegen wirkt. Gleichzeitig müssen die Stents in der Richtung quer zu ihrer Längserstreckung eine ausreichende Flexibilität aufweisen, um sich an gebogene Hohlorgane wie z.B. in Gelenkbereichen anpassen zu können, ohne dass die Gefahr des Abknickens des Stents und einer dadurch hervorgerufenen Verringerung oder Unterbrechung des Blutflusses in dem Blutgefäß oder gar eines Durchstoßens der Gefäßwand besteht.

Es sind bereits Stents vorgeschlagen worden, die mehrere Stützabschnitte und Verbindungs- bzw. Gelenkabschnitte umfassen, die in Längsrichtung des Stents aufeinander folgen. Die Stützabschnitte weisen dabei jeweils mehrere Durchbrechungen der Wand des röhrenförmigen Körpers und durch den röhrenförmigen Körper gebildete Umrandungselemente auf, die die Durchbrechungen umschließen und zusammen mit diesen im expandierten Zustand Zellen der Stützabschnitte bilden. Zwei in Längsrichtung benachbarte Stützabschnitte sind dabei über einen dazwischen liegenden Verbindungsabschnitt miteinander verbunden, wobei einander zugewandte Stirnseiten der benachbarten Stützabschnitte jeweils durch eine Reihe von stirnseitigen Zellen des jeweiligen Stützabschnitts gebildet sind. Der Verbindungsabschnitt umfasst ein oder mehrere durch den röhrenförmigen Körper gebildete Verbindungselemente, die die einander zugewandten Stirnseiten der zwei benachbarten Stützabschnitte miteinander verbinden.

Die Verbindungsabschnitte können bei diesen Stents als Gelenk zwischen den die radiale Aufstellkraft des Stents bestimmenden Stützabschnitten dienen und eine Anpassung der Stentform an eine gebogene Gefäßform erlauben, da benachbarte Stützabschnitte zueinander beweglich sind.

Aus der EP 0 832 618 A1 ist ein Stent gemäß dem Oberbegriff des Anspruchs 1 bekannt. Bei diesem Stent ist nur ein Teil der Zellen, die eine der zwei einander zugewandten Stirnseiten der benachbarten Stützabschnitte bilden, über ein Verbindungselement direkt mit der anderen der zwei einander zugewandten Stirnseiten der benachbarten Stützabschnitte verbunden.

Auch bei diesen Stents sind die gleichzeitig erreichbare radiale Aufstellkraft des Stents einerseits und dessen Flexibilität quer zur Längsrichtung andererseits derart begrenzt, dass sich diese Stents nicht für bestimmte Anwendungen eignen, die eine besonders hohe radiale Aufstellkraft und gleichzeitig eine besonders hohe Flexibilität erfordern. Beispielsweise besteht ein Bedarf an einem Stent, der sich für den Einsatz der insbesondere im Beckenbereich des Körpers befindlichen Venen eignet, wie z.B. der vena iliaca oder der vena femoralis. Ein derartiger Stent muss einem sehr hohen radialen Druck von der entsprechenden Gefäßwand standhalten und aufgrund der stark gebogenen Form der genannten Gefäße gleichzeitig eine außerordentlich hohe Flexibilität quer zu seiner Längsrichtung besitzen.

Der vorliegenden Erfindung liegt die Aufgabe zugrunde, einen Stent der eingangs genannten Art zu schaffen, der sich für den Einsatz in Hohlorganen eignet, deren Abstützung eine hohe radiale Aufstellkraft erfordert und die gleichzeitig stark gebogene bzw. gekrümmte Abschnitte umfassen, wie z.B. eine Vene im Beckenbereich des Körpers, wobei ein Einknicken oder Abknicken des Stents bei dessen Einsatz zuverlässig ausgeschlossen wird.

Diese Aufgabe wird erfindungsgemäß ausgehend von einem Stent der eingangs genannten Art durch die Merkmale des Anspruchs 1 gelöst. Der Stent umfasst wenigstens zwei in Längsrichtung benachbarte Stützabschnitte, die über einen dazwischenliegenden Verbindungsabschnitt miteinander verbunden sind. Dabei ist nur ein Teil der Zellen, die eine der zwei einander zugewandten Stirnseiten der zwei benachbarten Stützabschnitte bilden, über ein Verbindungselement direkt mit der anderen der zwei einander zugewandten Stirnseiten der benachbarten Stützabschnitte verbunden.

Dadurch, dass nur ein Teil der Zellen der einander zugewandten Stirnseiten der Stützabschnitte durch ein Verbindungselement des Verbindungsabschnitts mit der jeweils anderen Stirnseite direkt verbunden ist, werden die Flexibilität des Stents quer zur Längsrichtung und gleichzeitig dessen radiale Aufstellkraft erheblich gesteigert, da durch die nicht mit dem jeweils anderen Stützabschnitt verbundenen Zellen zwar eine zusätzliche Stützwirkung des Stützabschnitts bewirkt wird, gleichzeitig aber keine die Flexibilität herabsetzende Versteifung zwischen den Stützabschnitten hervorgerufen wird.

Erfindungsgemäß wurde erkannt, dass die Öffnungen in dem Verbindungsabschnitt, die infolge des Fehlens von direkten Verbindungen zwischen einem Teil der stirnseitigen Zellen der benachbarten Stützabschnitte im expandierten Stentzustand vergleichsweise groß sein können, nicht zu einer Verringerung der Verträglichkeit des in das Hohlorgan eingesetzten Stents führen. Insbesondere wurde erkannt, dass bei dem Einsatz eines solchen Stents in einer Vene, etwa im Beckenbereich des Körpers, größere Öffnungen in den Verbindungsabschnitten ohne Weiteres toleriert werden können, ohne dass die Gefahr einer Restenose oder anderer Unverträglichkeiten besteht. In den Stützabschnitten kann demgegenüber durch die vergleichsweise geringe Ausdehnung der Durchbrechungen eine hohe Stützwirkung erzielt werden. Eine im Vergleich zu den Zellen der Stützabschnitte vergrößerte Ausdehnung der Öffnungen in den Verbindungsabschnitten wird daher im Rahmen der Erfindung bewusst in Kauf genommen, um eine erhöhte Flexibilität des Stents bei gleichzeitig hoher radialer Aufstellkraft zu erreichen.

Erfindungsgemäß umfasst der Stent einen an seinem längsseitigen Ende angeordneten endseitigen Stützabschnitt sowie einen sich an diesen endseitigen Stützabschnitt anschließenden mittleren Bereich des Stents, wobei der mittlere Bereich des Stents durch eine Vielzahl von in Längsrichtung benachbarten, durch Verbindungsabschnitte miteinander verbundenen Stützabschnitten gebildet ist, die jeweils genau eine Reihe von Zellen aufweisen, die in Umfangsrichtung des Stents aufeinander folgen. Weiterhin weist der endseitige Stützabschnitt mehrere in Längsrichtung des Stents aufeinander folgende Reihen von Zellen auf, wobei die Zellen einer Reihe jeweils in Umfangsrichtung des Stents aufeinander folgend angeordnet sind, so dass der endseitige Stützabschnitt eine größere axiale Länge und eine größere Anzahl von in Längsrichtung des Stents aufeinander folgenden Reihen von in Umfangsrichtung des Stents aufeinander folgenden Zellen aufweist als die im mittleren Bereich des Stents angeordneten Stützabschnitte.

Vorteilhafte Ausführungsformen der Erfindung sind den Unteransprüchen, der Beschreibung und den Figuren zu entnehmen.

Prinzipiell kann der Stent mehr als zwei Stützabschnitte und mehr als einen Verbindungsabschnitt umfassen, die in Längsrichtung des Stents aufeinander folgen, wobei ein Verbindungsabschnitt jeweils zwei in Längsrichtung zueinander benachbarte Stützabschnitte miteinander verbindet. Die Stützabschnitte, die Verbindungsabschnitte und die Verbindungen zwischen zwei benachbarten Stützabschnitten über einen Verbindungsabschnitt können dabei jeweils so ausgebildet sein, wie es vorstehend in Bezug auf die wenigstens zwei benachbarten Stützabschnitte und den dazwischen liegenden Verbindungsabschnitt beschrieben ist. Wenn in der vorliegenden Beschreibung die Ausgestaltung eines Stützabschnitts, eines Verbindungsabschnitts oder einer Verbindung zwischen zwei benachbarten Stützabschnitten über einen Verbindungsabschnitt beschrieben ist, können dementsprechend prinzipiell stets ein oder mehrere und vorzugsweise alle Stützabschnitte, Verbindungsabschnitte bzw. Verbindungen zwischen Stützabschnitten in der jeweils beschriebenen Weise ausgestaltet sein. Die Beschreibung der Stentstruktur bezieht sich, soweit nicht anders angegeben, prinzipiell auf den Stent bei gerader Orientierung, das heißt im nicht verbogenen Zustand. Soweit nicht anders angegeben, weist der Stent die jeweils beschriebene Struktur zumindest im komprimierten oder im expandierten Zustand auf und kann die Struktur insbesondere in beiden Zuständen aufweisen.

Erfindungsgemäß sind die stirnseitigen Zellen zumindest eines der zwei benachbarten Stützabschnitte nur zum Teil über ein Verbindungselement des Verbindungsabschnitts direkt mit der Stirnseite des anderen Stützabschnitts verbunden. Bevorzugt ist es, wenn die stirnseitigen Zellen beider miteinander verbundener Stützabschnitte nur zum Teil über ein Verbindungselement direkt mit der anderen Stirnseite verbunden sind.

Gemäß einer vorteilhaften Ausführungsform ist die Anzahl der Verbindungselemente des Verbindungsabschnitts geringer als die Anzahl derjenigen Zellen zumindest eines der benachbarten Stützabschnitte, die eine der zwei über die Verbindungselemente des Verbindungsabschnitts miteinander verbundenen und einander zugewandten Stirnseiten bilden. Bevorzugt ist die Anzahl der Verbindungselemente geringer als die jeweilige Anzahl von Zellen beider benachbarter Stützabschnitte, die die jeweilige der einander zugewandten Stirnseiten bilden. Die Anzahl der die Stirnseiten bildenden Zellen kann dabei für beide benachbarten Stützabschnitte gleich oder unterschiedlich sein. Durch eine solche im Vergleich zu der Anzahl der stirnseitigen Zellen geringe Anzahl von Verbindungselementen wird eine entsprechend geringe Steifigkeit des Stents quer zur Längsrichtung und folglich eine hohe Flexibilität des Stents erreicht. Bevorzugt sind die stirnseitigen Zellen eines und bevorzugt beider benachbarter Stützabschnitte jeweils über maximal ein Verbindungselement des Verbindungsabschnitts direkt mit dem jeweils anderen Stützabschnitt verbunden.

Bevorzugt umfasst zumindest einer der beiden benachbarten Stützabschnitte eine Reihe von Zellen, die in Umfangsrichtung des Stents aufeinander folgen und vorzugsweise einen geschlossenen, in Umfangsrichtung des Stents umlaufenden Ring bilden. Der Ring weist senkrecht zur Längsrichtung des Stents vorzugsweise einen durch die Zellen des Rings gebildeten ringförmig geschlossenen Querschnitt auf. Durch einen solchen in Umfangsrichtung umlaufenden und bevorzugt geschlossenen Ring kann in dem Stützabschnitt eine optimale radiale Aufstellkraft und Stützwirkung des Stents geschaffen werden. Ein Ring kann prinzipiell durch beliebige Zellen des jeweiligen Stützabschnitts gebildet sein. Dabei decken die Zellen, die den Ring bilden, jeweils einen Bereich des Umfangs des Stents ab, wobei sich Umfangsbereiche, die von zumindest einem Teil und insbesondere allen Zellen des Rings abgedeckt werden, vorzugsweise nicht oder allenfalls unwesentlich überlappen oder miteinander decken. In Längsrichtung betrachtet sind die Zellen folglich zumindest annähernd vollständig nebeneinander und nicht hintereinander angeordnet. Ferner decken die Zellen, die den Ring bilden, jeweils einen Längenbereich des Stents ab, wobei sich die Längenbereiche, die von einem Teil und insbesondere allen Zellen des Rings abgedeckt werden, vorzugsweise zumindest annähernd vollständig überlappen bzw. miteinander decken, d.h. dass ein Längenbereich einen jeweiligen anderen Längenbereich zumindest annähernd vollständig umfasst oder von diesem umfasst wird. In Umfangsrichtung betrachtet sind die Zellen folglich zumindest annähernd vollständig hintereinander und nicht nebeneinander angeordnet. Unter einer unwesentlichen Überlappung wird in dieser Beschreibung prinzipiell eine Überlappung von bis zu maximal 20 %, bevorzugt bis zu 10 % oder bis zu 5 % verstanden und unter einer zumindest annähernd vollständigen Überlappung eine Überlappung von zumindest 80 %, bevorzugt zumindest 90 % oder zumindest 95 %.

Wie nachstehend erläutert, kann ein Stützabschnitt mehrere in Längsrichtung des Stents aufeinander folgende Reihen von Zellen umfassen, die jeweils einen wie vorstehend beschriebenen Ring bilden. Ein Stützabschnitt kann auch aus genau einer Reihe von Zellen bestehen, die in Umfangsrichtung des Stents aufeinander folgen und bevorzugt einen wie vorstehend beschrieben Ring bilden. Insbesondere bei der letztgenannten Ausgestaltung können beide längsseitigen Stirnseiten des jeweiligen Stützabschnitts, d.h. sowohl eine vordere als auch eine hintere längsseitige Stirnseite des Stützabschnitts, ganz oder teilweise durch dieselben Zellen des Stützabschnitts gebildet sein. Prinzipiell ist es bevorzugt, wenn ein wie vorstehend beschriebener Ring zumindest teilweise und vorzugsweise vollständig aus Zellen besteht, die zumindest eine der einander zugewandten Stirnseiten zweier benachbarter Stützabschnitte bilden.

Gemäß einer Ausführungsform weisen eine oder mehrere stirnseitige Zellen eines Stützabschnitts jeweils ein freies Ende auf, das dem jeweils benachbarten Stützabschnitt bzw. dessen Stirnseite zugewandt ist. Das freie Ende dieser Zellen weist vorzugsweise eine abgerundete Form auf, beispielsweise die Form einer abgerundeten Spitze, welche eine atraumatische Rundung darstellt. Durch die Ausbildung des freien Endes einer Zelle mit einer solchen atraumatischen Rundung kann eine Verletzung des mit dem Stent abgestützten Hohlorgans selbst dann zuverlässig vermieden werden, wenn die freien Enden des Stents bei einer starken Verbiegung des Stents geringfügig nach außen hervorstehen und in die Wand des Hohlorgans eingedrückt werden. Die Rundung kann beispielsweise in etwa kreissegmentförmig und insbesondere im Wesentlichen halbkreisförmig ausgestaltet sein.

Gemäß einer weiteren vorteilhaften Ausführungsform sind die einander zugewandten stirnseitigen Zellen zweier benachbarter Stützabschnitte in Umfangsrichtung des Stents zueinander versetzt angeordnet. Das freie Ende einer stirnseitigen Zelle eines Stützabschnitts kann dabei in Bezug auf seine Position in Umfangsrichtung zwischen den freien Enden zweier in Umfangsrichtung aufeinander folgender stirnseitiger Zellen des anderen Stützabschnitts angeordnet sein. Bevorzugt sind die freien Enden mehrerer und insbesondere aller stirnseitiger Zellen eines Stützabschnitts in der vorstehend beschriebenen Weise so angeordnet, dass sie in Bezug auf ihre Position in Umfangsrichtung zwischen den freien Enden zweier in Umfangsrichtung aufeinander folgender stirnseitiger Zellen des anderen Stützabschnitts angeordnet sind.

Durch den vorstehend beschriebenen Versatz der benachbarten Stützabschnitte in Umfangsrichtung kann vermieden werden, dass bei einer starken Verbiegung des Stents die freien Enden der einander zugewandten Stirnseiten der benachbarten Stützabschnitte miteinander bzw. mit der jeweils gegenüberliegenden Stirnseite kollidieren. Die Gefahr einer solchen Kollision bestünde andernfalls bei einer starken Verbiegung bzw. Krümmung des Stents quer zur Längsrichtung, da sich dabei die krümmungsinnenseitig gelegenen stirnseitigen Zellen der Stützabschnitte in Längsrichtung des Stents aufeinander zu bewegen. Aufgrund der versetzten Anordnung der Zellen der Stützabschnitte kann das freie Ende einer Zelle eines Stützabschnitts in den Zwischenraum zwischen zwei freien Enden des anderen Stützabschnitts einfahren, wenn sich die freien Enden der Stützabschnitte infolge einer Verbiegung des Stents aufeinander zu bewegen. Eine besonders günstige Ausgestaltung ergibt sich, wenn das freie Ende einer oder mehrerer und insbesondere aller stirnseitiger Zellen eines Stützabschnitts bezogen auf seine Position in Umfangsrichtung zumindest annähernd in der Mitte zwischen zwei in Umfangsrichtung aufeinander folgenden stirnseitigen Zellen des anderen Stützabschnitts angeordnet ist bzw. wenn die stirnseitigen Zellen der benachbarten Stützabschnitte um zumindest annähernd eine halbe, in Umfangsrichtung gemessene Zellenbreite zueinander versetzt angeordnet sind.

Gemäß einer Ausführungsform sind zumindest eine oder mehrere und insbesondere alle Zellen eines Stützabschnitts im expandierten Zustand des Stents im Wesentlichen rautenförmig ausgebildet. Im komprimierten Zustand des Stents kann eine solche Zelle beispielsweise durch einen im Wesentlichen geraden und vorzugsweise in Längsrichtung des Stents orientierten Schlitz des röhrenförmigen Körpers und die den Schlitz umgebenden Umrandungselemente gebildet sein. Diese Schlitze können im Zuge der Expansion des Stents zu den rautenförmigen Durchbrechungen aufgeweitet werden. Die Rauten des expandierten Stents können in Längsrichtung des Stents langgestreckt ausgebildet sein, das heißt eine größere Länge als Breite aufweisen. Im expandierten Zustand können die die Rauten definierenden Umrandungselemente der Zellen zumindest näherungsweise gerade ausgebildet sein und beispielsweise jeweils in einem Winkel zwischen zwanzig und vierzig Grad und vorzugsweise etwa dreißig Grad zu der Längsachse des Stents geneigt orientiert sein. Mit derartigen rautenförmigen Zellen lässt sich eine besonders hohe radiale Aufstellkraft und Stützwirkung eines Stützabschnitts erreichen.

Gemäß einer weiteren Ausführungsform weisen zumindest eine oder mehrere und insbesondere alle Zellen eines Stützabschnitts im expandierten Zustand des Stents zumindest einen und vorzugsweise drei schräg zur Längsachse des Stents angeordnete Abschnitte auf. Derartige Zellen gewährleisten eine erhöhte Flexibilität und Biegbarkeit des Stützabschnitts quer zur Längsrichtung bei gleichzeitig guter radialer Aufstellkraft. Zusätzlich zu der durch die Verbindungsabschnitte geschaffenen hohen Flexibilität weist bei einer solchen Ausgestaltung der Zellen auch der Stützabschnitt selbst eine relativ hohe Flexibilität quer zur Längsrichtung des Stents auf, so dass die Flexibilität des Stents bei gleichzeitiger Aufrechterhaltung einer hohen Aufstellkraft und Stützwirkung noch weiter gesteigert werden kann. Die beschriebenen Zellen können im komprimierten Zustand des Stents durch ein oder mehrere und insbesondere drei schräg zur Längsachse des Stents orientierte Schlitze gebildet sein. Die Zellen können im Wesentlichen wellenförmig, insbesondere sinuswellenförmig ausgebildet sein.

Prinzipiell ist es im Rahmen der Erfindung bevorzugt, wenn ein Stützabschnitt zumindest teilweise und insbesondere vollständig durch geschlossene Zellen gebildet ist. Eine geschlossene Zelle kann im komprimierten Zustand des Stents durch eine Durchbrechung gebildet sein, die bezogen auf die Längsrichtung des Stents vorzugsweise umkehrungsfrei in einer Richtung verläuft und insbesondere keine in Umfangsrichtung des Stents voneinander beabstandeten Teilschlitze bzw. Teildurchbrechungen bildet, die in Umfangsrichtung überlappend hintereinander angeordnet sind, d.h. die sich deckende bzw. miteinander überlappende Längenbereiche des Stents abdecken. Die Durchbrechung bildet folglich im komprimierten Zustand des Stents vorzugsweise keine Hinterschneidung in Umfangsrichtung des Stents. Dementsprechend kann die Durchbrechung im expandierten Zustand ebenfalls eine geschlossene Fläche ohne eine Hinterschneidung in Umfangsrichtung des Stents bilden. In dem komprimierten Zustand des Stents können die Durchbrechungen der geschlossenen Zellen im Wesentlichen vollständig schlitzförmig ohne flächige Ausschnitte bzw. fensterförmige Öffnungen ausgebildet sein. Die Verwendung derartiger geschlossener Zellen führt zu einer besonders hohen radialen Aufstellkraft und Stützwirkung des Stents in den Stützabschnitten.

Die ein oder mehreren Verbindungselemente der Verbindungsabschnitte sind vorzugsweise derart ausgebildet, dass sie eine erhöhte Flexibilität quer zur Längsrichtung des Stents aufweisen und sich vergleichsweise leicht verbiegen lassen und somit dem Stent eine hohe Flexibilität verleihen. Die Verbindungselemente sind durch den röhrenförmigen Körper gebildet und können beispielsweise durch einzelne lang gestreckte stegförmige Abschnitte des Körpers gebildet sein, ebenso wie die Umrandungselemente der Zellen eines Stützabschnitts. Die Breite der Verbindungselemente kann dabei zumindest abschnittsweise und zumindest näherungsweise der Breite der Umrandungselemente entsprechen.

Gemäß einer Ausführungsform weisen zumindest ein oder mehrere und insbesondere alle Verbindungselemente eines Verbindungsabschnitts eine in Längsrichtung des Stents gemessene Länge auf, die zumindest die Hälfte und bevorzugt zumindest drei Viertel der in Längsrichtung des Stents gemessenen maximalen Länge der durch das Verbindungselement verbundenen stirnseitigen Zellen der Stützabschnitte beträgt. Die Länge der Verbindungselemente kann auch zumindest annähernd so groß oder größer sein als die Länge dieser Zellen. Allgemein kann die Länge des Verbindungselements zumindest 50 %, zumindest 75 %, zumindest 100 % oder mehr der maximalen Länge zumindest einer und vorzugsweise aller stirnseitigen Zellen eines oder beider benachbarter Stützabschnitte betragen. Durch diese verhältnismäßig große Längserstreckung im Vergleich zu den Zellen der Stützabschnitte wird eine besonders hohe Flexibilität der Verbindungselemente quer zur Längsrichtung des Stents erreicht.

Ein oder mehrere und insbesondere alle Verbindungselemente eines Verbindungsabschnitts können in Bezug auf die Längsachse des Stents einen relativ geringen, auf die gedachte Verbindungslinie zwischen ihrem Anfangs- und ihrem Endpunkt bezogenen Neigungswinkel von - im expandierten Stentzustand - beispielsweise zwischen fünf und fünfundzwanzig und bevorzugt etwa zehn Grad aufweisen. Dabei kann der in Umfangsrichtung gemessene, vorzugsweise relativ geringfügige Versatz zwischen dem Anfangs- und dem Endpunkt eines jeweiligen Verbindungselements beispielsweise bis zu einer in Umfangsrichtung des Stents gemessenen halben Zellenbreite der Zellen der Stützabschnitte oder bis zu näherungsweise der eineinhalbfachen Zellenbreite betragen. Dadurch wird zum einen eine hohe Flexibilität des Stents in allen Richtungen senkrecht zur Längsachse des Stents und gleichzeitig eine hohe Stabilität und Zugfestigkeit in Längsrichtung des Stents gewährleistet.

Wie vorstehend beschrieben, können die stirnseitigen Zellen der Stützabschnitte in Richtung des jeweils benachbarten Stützabschnitts weisende freie Enden aufweisen, die z.B. abgerundet ausgebildet und zu den freien Enden der Zellen des anderen Stützabschnitts versetzt angeordnet sein können. Vorzugsweise weisen insbesondere auch zumindest ein oder mehrere und insbesondere alle diejenigen stirnseitigen Zellen der benachbarten Stützabschnitte ein solches freies Ende auf, die über ein Verbindungselement direkt mit dem jeweils anderen Stützabschnitt verbunden sind. Ein Verbindungselement kann dabei in einem Bereich mit einer stirnseitigen Zelle eines Stützabschnitts verbunden sein, der von einem dem jeweils anderen Stützabschnitt zugewandten freien Ende dieser Zelle, bevorzugt in Umfangsrichtung des Stents, beabstandet ist und gegenüber dem freien Ende der Zelle bezogen auf die Längsrichtung des Stents vorzugsweise nach hinten zurückversetzt angeordnet ist. Ein Verbindungselement kann dabei zumindest näherungsweise in einem Bereich der Stirnseite mit der Stirnseite verbunden sein, welcher bezogen auf die Längsrichtung des Stents am weitesten nach hinten zurückversetzt angeordnet ist. Ebenso kann das Verbindungselement in Umfangsrichtung zumindest näherungsweise in der Mitte zwischen den freien Enden zweier in Umfangsrichtung aufeinander folgender stirnseitiger Zellen eines Stützabschnitts angeordnet sein. Dadurch wird eine längere Ausgestaltung des Verbindungselements ermöglicht, ohne dass eine Vergrößerung des Abstands zwischen den beiden Stützabschnitten insgesamt notwendig ist. Dabei wird infolge des kurzen Abstands zwischen den benachbarten Stützabschnitten eine sehr gute Stützwirkung bei gleichzeitig hoher Flexibilität des Stents erreicht.

Gemäß einer Ausführungsform ist zumindest ein Verbindungselement des Verbindungsabschnitts in einem Bereich mit der Stirnseite eines Stützabschnitts verbunden, in dem zwei die Stirnseite bildende, zueinander benachbarte Zellen dieses Stützabschnitts miteinander verbunden sind. Ein solcher Verbindungsbereich ist in Umfangsrichtung betrachtet typischerweise in der Mitte zwischen den freien Enden der beiden Zellen angeordnet und erlaubt eine besonders lange und damit flexible Ausgestaltung des jeweiligen Verbindungselements, ohne dass der Abstand zwischen den beiden Stützabschnitten erhöht wird. Das Verbindungselement kann sich bei einer solchen Verbindung zumindest über einen Teil seiner Länge in Umfangsrichtung zwischen zwei stirnseitigen Zellen eines Stützabschnitts befinden. Um insbesondere im komprimierten Zustand des Stents den für das Verbindungselement notwendigen Raum zu schaffen, kann der Verbindungsbereich, mit dem das Verbindungselement verbunden ist, gegenüber einem entsprechenden Verbindungsbereich zwischen zwei stirnseitigen Zellen des Stützabschnitts in Umfangsrichtung geringfügig, insbesondere in etwa um die Breite des Verbindungselements, verbreitert bzw. erweitert ausgebildet sein. Auf der dem Verbindungselement abgewandten Seite des Verbindungsbereichs kann dabei entweder ein weiteres Verbindungselement angeordnet sein, insbesondere wenn der jeweilige Stützabschnitt aus genau einem Ring von Zellen besteht, oder es kann dort eine Durchbrechung ausgebildet sein, die gegenüber einem reinen Schlitz erweitert ist und eine Breite aufweist, die insbesondere im Wesentlichen der Breite des Verbindungselements entspricht.

Die Verbindungsabschnitte umfassen jeweils eine oder mehrere durchgehende Öffnungen des röhrenförmigen Körpers, die durch die ein oder mehreren Verbindungselemente begrenzt werden. Eine Öffnung des Verbindungsabschnitts wird vorzugsweise durch ein oder mehrere Verbindungselemente sowie durch Abschnitte zumindest eines und vorzugsweise beider der einander zugewandten Stirnseiten der durch den Verbindungsabschnitt miteinander verbundenen Stützabschnitte umrandet und umschlossen.

Vorzugsweise umfasst der Verbindungsabschnitt mehrere Öffnungen, die eine senkrecht zur Längsrichtung orientierte Ebene definieren, welche mehrere und vorzugsweise alle Öffnungen des Verbindungsabschnitts schneidet.

Die Öffnungen des Verbindungsabschnitts decken dabei jeweils einen Bereich des Umfangs des Stents ab, wobei sich zumindest ein Teil und insbesondere alle Umfangsbereiche, die von den Öffnungen abgedeckt werden, vorzugsweise nicht ober allenfalls unwesentlich überlappen bzw. miteinander decken. In Längsrichtung sind die Öffnungen folglich zumindest annähernd vollständig nebeneinander und nicht hintereinander angeordnet. Ferner decken die Öffnungen des Verbindungsabschnitts jeweils einen Längenbereich des Stents ab, wobei sich zumindest ein Teil und vorzugsweise alle Längenbereiche, die von den Öffnungen abgedeckt werden, vorzugsweise zumindest annähernd vollständig überlappen bzw. miteinander decken, d.h. dass ein Längenbereich einen jeweiligen anderen Längenbereich zumindest annähernd vollständig umfasst. In Umfangsrichtung betrachtet sind die Öffnungen folglich zumindest annähernd vollständig hintereinander und nicht nebeneinander angeordnet. Die Öffnungen können also rein in Umfangsrichtung aufeinander folgen und in Längsrichtung ganz oder teilweise hintereinander angeordnete Öffnungen werden bei dieser Ausgestaltung vermieden, wodurch eine hohe Flexibilität bei geringer Länge des Verbindungsabschnitts erreicht wird.

Entsprechend der vorzugsweise relativ geringen Anzahl von Verbindungselementen des Verbindungsabschnitts ist die Anzahl der Öffnungen des Verbindungsabschnitts bevorzugt geringer als die Anzahl der Zellen, die jeweils eine der einander zugewandten Stirnseiten der beiden durch den Verbindungsabschnitt verbundenen Stützabschnitte bilden. Dadurch wird eine hohe Flexibilität des Verbindungsabschnitts gewährleistet. In einem besonders einfachen Fall kann ein Verbindungsabschnitt genau ein Verbindungselement umfassen, wobei der Verbindungsabschnitt genau eine sich in Umfangsrichtung um den Stent herum erstreckende Öffnung umfassen kann, die in Umfangsrichtung betrachtet beidseitig durch das eine Verbindungselement begrenzt ist.

Die Flexibilität der Verbindungsabschnitte lässt sich dadurch erhöhen, dass eine oder mehrere und insbesondere alle Öffnungen der Verbindungsabschnitte im komprimierten Zustand des Stents nicht rein schlitzförmig, sondern zumindest bereichsweise flächig ausgestaltet sind und einen fensterartigen Ausschnitt bilden. Eine oder mehrere und insbesondere alle Öffnungen des Verbindungsabschnitts können zumindest im expandierten Zustand des Stents eine Fläche aufweisen, die größer ist als die jeweilige Fläche einer oder mehrerer und insbesondere aller Durchbrechungen, die zu den stirnseitigen Zellen der benachbarten Stützabschnitte gehören. Die Fläche einer Öffnung des Verbindungsabschnitts kann dabei um zumindest 50 %, vorzugsweise zumindest 100 % und besonders bevorzugt zumindest 200 % größer sein als die jeweilige Fläche der Durchbrechungen der Stützabschnitte. Die Fläche der Öffnungen bzw. Durchbrechungen ist dabei als die Teilfläche der äußeren einhüllenden Mantelfläche des Stents definiert, der durch die jeweilige Öffnung bzw. Durchbrechung gebildet ist.

Gemäß einer vorteilhaften Ausführungsform sind zumindest ein und bevorzugt mehrere und insbesondere alle Verbindungselemente eines Verbindungsabschnitts zumindest abschnittsweise quer zur Längsrichtung des Stents orientiert. Auf diese Weise lässt sich zum einen die Flexibilität des Verbindungselements quer zur Längsrichtung des Stents erhöhen und zum anderen ein wie vorstehend beschriebener Versatz zwischen den Zellen der benachbarten Stützabschnitte in Umfangsrichtung überbrücken.

Ein oder mehrere und insbesondere alle Verbindungselemente eines Verbindungsabschnitts können eine geknickte oder gebogene Form aufweisen und vorzugsweise im Wesentlichen S- oder Z-förmig ausgebildet sein. Eine solche Form gewährleistet eine besonders hohe Flexibilität der Verbindungselemente quer zu der Längsrichtung des Stents.

Gemäß der Erfindung weist ein Stützabschnitt des mittleren Bereichs des Stents genau eine Reihe von Zellen auf, die in Umfangsrichtung des Stents aufeinander folgen. Die Reihe von Zellen kann dabei einen wie vorstehenden beschriebenen geschlossenen Ring des Stützabschnitts bilden. Die Zellen der einen Reihe können dabei sowohl eine vordere als auch eine hintere längsseitige Stirnseite des Stützabschnitts bilden. Mit einem solchen Stützabschnitt lässt sich eine gute Aufstellwirkung erzielen, wobei eine Vielzahl von derartigen, jeweils über einen Verbindungsabschnitt miteinander verbundenen Stützabschnitten eine besonders hohe Flexibilität des Stents gewährleistet. Zumindest ein endseitiger Stützabschnitt weist mehrere in Längsrichtung des Stents aufeinander folgende Reihen von Zellen auf, wobei die Zellen einer Reihe jeweils in Umfangsrichtung des Stents aufeinander folgend angeordnet sind. Die Reihen von Zellen bilden dabei zumindest im expandierten Zustand des Stents vorzugsweise ein Gitter bzw. Gitternetz aus sich sowohl in Längsrichtung als auch in Umfangsrichtung des Stents wiederholenden Zellen. Eine Reihe von in Umfangsrichtung aufeinander folgenden Zellen des Stützabschnitts kann dabei jeweils einen wie vorstehend bereits beschriebenen, in Umfangsrichtung des Stents umlaufenden geschlossenen Ring bilden.

Gemäß einer vorteilhaften Ausführungsform weist der Stent mehr als zwei Stützabschnitte und mehrere Verbindungsabschnitte auf, die in Längsrichtung des Stents aufeinander folgen, wobei ein Verbindungsabschnitt jeweils zwei zueinander benachbarte und in Längsrichtung aufeinander folgende Stützabschnitte miteinander verbindet. Durch das Vorsehen mehrerer Stützabschnitte und Verbindungsabschnitte, die jeweils zwei Stützabschnitte verbinden, wird die Flexibilität des Stents verbessert. Prinzipiell ist es bevorzugt, wenn der Stent zumindest drei Stützabschnitte aufweist, wobei sich ein Stützabschnitt in Längsrichtung des Stents betrachtet in der Mitte des Stents befindet, während die anderen beiden Stützabschnitte beispielsweise an den längsseitigen Enden des Stents angeordnet sein können. Der Stent kann aber auch zumindest vier, acht, zehn, zwanzig oder bis zu vierzig und mehr Stützabschnitte und eine entsprechende Anzahl von Verbindungsabschnitten aufweisen, die in Längsrichtung des Stents aufeinander folgen.

Bevorzugt sind die Verbindungselemente zweier insbesondere in Längsrichtung aufeinander folgender und zueinander benachbarter Verbindungsabschnitte in Umfangsrichtung des Stents zueinander versetzt angeordnet. Dadurch lässt sich eine gleichmäßige Flexibilität des Stents in alle Richtungen senkrecht zur Längsrichtung erreichen.

Gemäß der Erfindung weist der endseitige Stützabschnitt eine größere in Längsrichtung des Stents gemessene Länge auf als die im mittleren Bereich des Stents angeordneten Stützabschnitte. Zusätzlich weist der endseitige Stützabschnitt eine größere Anzahl von in Längsrichtung des Stents aufeinander folgenden Reihen von in Umfangsrichtung des Stents aufeinander folgenden Zellen auf als der im mittleren Bereich des Stents angeordnete Stützabschnitt. Durch die Verwendung von Stützabschnitten mit unterschiedlichen Längen und unterschiedlichen Anzahlen von in Längsrichtung aufeinander folgenden Zellenreihen kann die Steifigkeit des Stents in den entsprechenden Bereichen gezielt erhöht oder erniedrigt werden, z.B. um ein gewünschtes Verhalten beim Einführen des Stents zu erreichen. Erfindungsgemäß weist ein Stützabschnitt, der an einem längsseitigen Ende des Stents angeordnet ist, eine größere axiale Länge und/oder eine größere Anzahl von Zellenreihen auf als ein in einem mittleren Bereich des Stents angeordneter Stützabschnitt, um dadurch eine größere Stabilität bzw. Steifigkeit des Stents an dem längsseitigen Ende zu erreichen.

Ein Verbindungsabschnitt des Stents kann beispielsweise zwischen einem und zehn Verbindungselementen und bevorzugt ein, zwei, drei oder vier Verbindungselemente aufweisen. Eine derartige, relativ geringe Anzahl von Verbindungselementen erweist sich als vorteilhaft, um die gewünschte hohe Flexibilität des Stents senkrecht zu der Längsrichtung des Stents zu erreichen.

Bevorzugt umfasst ein Verbindungsabschnitt mehrere Verbindungselemente, die in zumindest näherungsweise gleichen Winkelabständen über den Umfang des Stents verteilt angeordnet sind, wobei die Winkelabstände sich auf den bevorzugt in etwa kreisförmigen Querschnitt des Stents beziehen.

Die Stirnseite eines Stützabschnitts kann beispielsweise durch 4 bis 42, vorzugsweise zwischen 8 und 34 und besonders bevorzugt zwischen 12 und 24 Zellen gebildet sein. Eine solche Anzahl von Zellen erweist sich als vorteilhaft, um die gewünschte hohe radiale Aufstellkraft und Stützwirkung des Stents in den Stützabschnitten zu erreichen. Diese Anzahlen sind auch in Bezug auf einen durch einen Stützabschnitt gebildeten und in Umfangsrichtung um den Stent umlaufenden Ring von Zellen bevorzugt.

Eine besonders hohe Stützwirkung des Stents lässt sich erreichen, wenn der im Wesentlichen röhrenförmige Körper des Stents eine Wandstärke von zwischen 0,20 mm und 0,40 mm, bevorzugt zwischen 0,28 mm und 0,45 mm und besonders bevorzugt zwischen 0,30 mm und 0,50 mm aufweist. Der Durchmesser des Stents im expandierten Zustand kann vorzugsweise zwischen 6 und 30 mm oder zwischen 10 und 24 mm betragen. Die Länge des Stents im expandierten Zustand kann zwischen 30 und 250 mm und bevorzugt zwischen 50 und 180 mm betragen.

Der Stent ist bevorzugt als selbst expandierender Stent ausgebildet und kann vorzugsweise ein Formgedächtnismaterial wie z.B. Nitinol umfassen oder daraus bestehen.

Der Stent kann zumindest an einem seiner längsseitigen Enden ein oder mehrere Halteelemente umfassen, die einen sich an den restlichen Stent anschließenden Halsabschnitt und einen über den Halsabschnitt mit dem restlichen Stent verbundenen, gegenüber dem Halsabschnitt verbreiterten Halteabschnitt aufweisen. Diese Halteelemente können mit entsprechenden Positionierelementen eines Einführkatheters zusammenwirken, um den Stent bis zu seiner Freigabe in der gewünschten Position relativ zu dem Einführkatheter zu halten. Ebenso kann der Stent an einem oder beiden längsseitigen Enden ein oder mehrere flächige Markierelemente aufweisen, die die Röntgenbeobachtbarkeit des Stents verbessern. Die Halteelemente und Markierelemente sind vorzugsweise durch den röhrenförmigen Körper des Stents gebildet.

Nachfolgend wird die Erfindung anhand vorteilhafter Ausführungsformen unter Bezugnahme auf die beigefügten Figuren beschrieben. Es zeigen:
- Fig. 1: einen Stent im komprimierten Zustand und in einer in eine Ebene projizierten Darstellung,
- Fig. 2: einen Stent im expandierten Zustand in Seitenansicht,
- Fig. 3: eine Zelle des Stents von Fig. 2,
- Fig. 4: einen Stent gemäß einer Ausführungsform der Erfindung im expandierten Zustand in Seitenansicht,
- Fig. 5: einen Stent im expandierten Zustand in Seitenansicht, und
- Fig. 6: einen Stent gemäß einer weiteren Ausführungsform der Erfindung im expandierten Zustand in Seitenansicht.

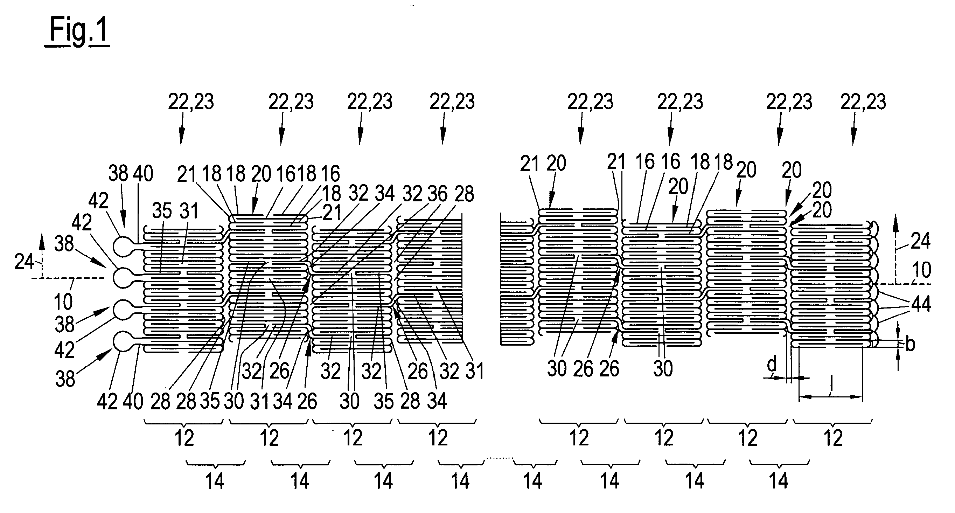

Fig. 1 zeigt einen Stent im komprimierten Zustand und in einer in eine Ebene projizierten bzw. in Umfangsrichtung "abgerollten" Darstellung.

Der durch einen röhrenförmigen Körper gebildete Stent ist entlang seiner Längsachse 10 in mehrere verschiedene Längsabschnitte 12, 14 unterteilt. Der Stent umfasst mehrere Stützabschnitte 12 und Verbindungsabschnitte 14, wobei jeweils zwei in Längsrichtung zueinander benachbarte Stützabschnitte 12 durch einen dazwischen liegenden Verbindungsabschnitt 14 miteinander verbunden sind.

Die Stützabschnitte 12 umfassen jeweils mehrere, in Längsrichtung 10 orientierte gerade und im komprimierten Zustand schlitzförmige Durchbrechungen 16 und durch den röhrenförmigen Körper gebildete Umrandungselemente 18, die die Durchbrechungen 16 umschließen und zusammen mit diesen Zellen 20 der Stützabschnitte 12 bilden. Die Zellen 20 weisen freie Enden 21 auf, die jeweils zur Bildung einer atraumatischen Rundung abgerundet ausgebildet sind.

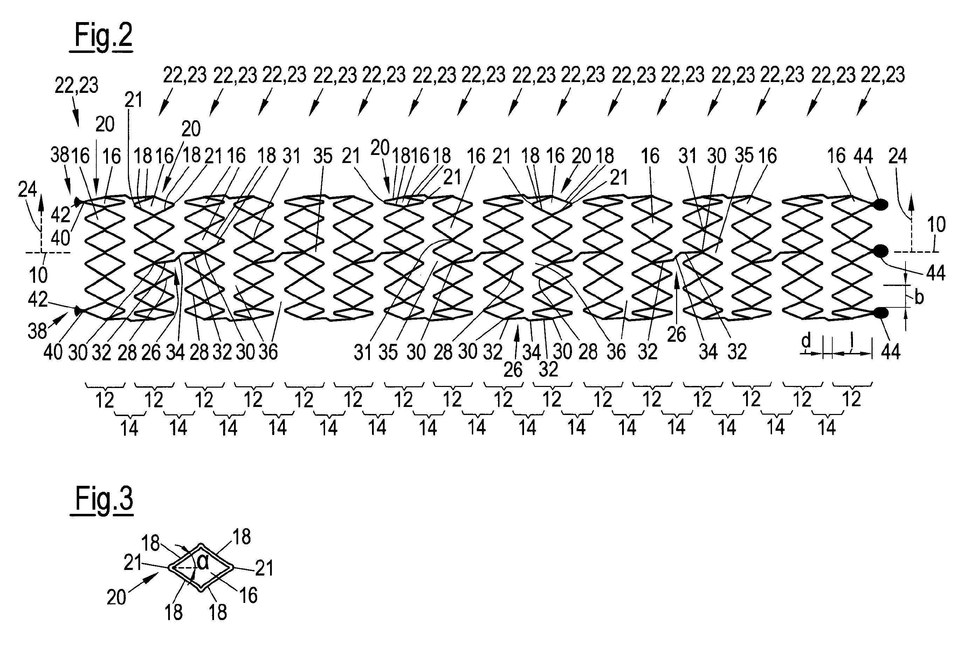

Jeder Stützabschnitt 12 besteht aus einer Reihe 22 von Zellen 20, die in Umfangsrichtung 24 des Stents aufeinander folgen und sich in Umfangsrichtung 24 betrachtet zumindest näherungsweise vollständig überlappen, ohne sich in Längsrichtung 10 des Stents betrachtet zu überlappen. Die von den Zellen 20 einer Reihe 22 jeweils abgedeckten Längenbereiche überlappen also im Wesentlichen vollständig miteinander und decken sich, während die von den Zellen 20 der Reihe 22 abgedeckten Umfangsbereiche nicht überlappen oder sich decken. Die Reihen 22 von Zellen 20 bilden dabei jeweils einen in Umfangsrichtung 24 um den Stent umlaufenden geschlossenen Ring 23. Die Zellen 20 benachbarter Stützabschnitte 12 sind um die Hälfte einer Zellenbreite b (siehe auch Fig. 2) in Umfangsrichtung 24 des Stents zueinander versetzt angeordnet, so dass die freien Enden 21 bei einer Verbiegung des Stents senkrecht zur Längsachse 10 nicht kollidieren.

Die Verbindungsabschnitte 14 umfassen jeweils zwei durch den röhrenförmigen Körper des Stents gebildete, im Wesentlichen S-förmige Verbindungselemente 26, die jeweils die einander zugewandten Stirnseiten 28 der zwei durch den jeweiligen Verbindungsabschnitt 14 miteinander verbundenen Stützabschnitte 12 miteinander verbinden. Die Zellen 20 eines Stützabschnitts 12 bilden im vorliegenden Ausführungsbeispiel sowohl die vordere als auch die hintere Stirnseite 28 eines Stützabschnitts 12, da nur eine Reihe 22 von Zellen 20 vorhanden ist. Wie in Fig. 1 ersichtlich, sind die Verbindungselemente 26 eines Verbindungsabschnitts 14 gleichmäßig über den Umfang des Stents verteilt, d.h. im vorliegenden Ausführungsbeispiel mit zwei Verbindungselementen 26 pro Verbindungsabschnitt 14 in einem Winkelabstand von 180 Grad zueinander.

Die Verbindungselemente 26 sind jeweils in einem Bereich 30 der Stirnseiten 28 mit diesen verbunden, in dem zwei in Umfangsrichtung 24 aufeinander folgende Zellen 20 des jeweiligen Stützabschnitts 12 miteinander verbunden sind und ineinander übergehen. Die Bereiche 30 zweier benachbarter Stützabschnitte 12, in denen ein Verbindungselement 26 jeweils mit den Stützabschnitten 12 verbunden ist, sind in Umfangsrichtung 24 um eineinhalb Zellenbreiten b zueinander versetzt angeordnet. Fig. 2 zeigt demgegenüber eine Ausgestaltung, bei der die Bereiche 30 der Stützabschnitte 12, mit denen die beiden Enden eines Verbindungselements 26 verbunden sind, um eine halbe Zellenbreite b in Umfangsrichtung 24 zueinander versetzt sind.

Die Verbindungselemente 26 weisen, wie in Fig. 1 gezeigt, jeweils zwei im komprimierten Zustand des Stents in Längsrichtung 10 orientierte gerade Abschnitte 32 auf, die jeweils mit einem der Bereiche 30 verbunden sind, und einen zwischen den Abschnitten 32 angeordneten und gegenüber der Längsrichtung 10 geneigten Abschnitt 34, der eine Erstreckung in Umfangsrichtung 24 aufweist, die der 1,5-fachen Zellenbreite b entspricht und die somit dem Umfangsversatz zwischen den miteinander verbundenen Bereichen 30 entspricht. Die Abschnitte 32 erstrecken sich jeweils zwischen Umrandungselementen 18 der durch den Verbindungsbereich 30 verbundenen Zellen 20. Um den für die Abschnitte 32 erforderlichen Raum zu schaffen, sind die Verbindungsbereiche 30, die mit einem Verbindungselement 26 verbunden sind, gegenüber den Verbindungsbereichen 31 zweier in Umfangsrichtung 24 benachbarter Zellen 22, die nicht mit einem Verbindungselement 26 verbunden sind, in Umfangsrichtung 24 verbreitert ausgestaltet. Konkret sind die Bereiche 30 gegenüber den Bereichen 31 um die Breite des jeweiligen Abschnitts 32 des Verbindungselements 26 verbreitert, welche näherungsweise der Breite eines Umrandungselements 18, d.h. b/2 in Fig. 1, entspricht. Auf der dem Verbindungselement 26 abgewandten Seite des Verbindungsbereichs 30 könnte prinzipiell ein weiteres Verbindungselement 26 angeordnet sein. Im vorliegenden Ausführungsbeispiel sind an den von dem jeweiligen Verbindungselement 26 abgewandten Seiten der Verbindungsbereiche 30 flächig ausgeschnittene Bereiche 35 angeordnet, die sich im komprimierten Zustand des Stents in Längsrichtung 10 zwischen den Umrandungselementen 18 der benachbarten Zellen erstrecken und in etwa die Form eines Abschnitts 32 eines Verbindungselements 26 aufweisen, d.h. insbesondere in etwa die Breite eines Umrandungselements 18 aufweisen.

Wie in Fig. 2 gezeigt, sind in dem expandierten Zustand des Stents auch die im komprimierten Zustand parallel zu der Längsrichtung 10 orientierten Abschnitte 32 der Verbindungselemente 26 infolge der Expansion leicht gegenüber der Längsrichtung 10 geneigt.

Die beiden im komprimierten Stentzustand parallel zur Längsrichtung 10 orientierten Abschnitte 32 sind in Längsrichtung 10 betrachtet in etwa so lang wie eine halbe Zellenlänge 1, so dass sich zusammen mit dem mittleren Abschnitt 34 eine Gesamtlänge eines Verbindungselements 26 ergibt, die etwas größer als die Zellenlänge 1 ist und einen Abstand d zwischen den beiden benachbarten Stützabschnitten 12 überbrückt. Zwischen den Verbindungselementen 26 sind, wie in Fig. 1 gezeigt, Öffnungen 36 des Verbindungsabschnitts 14 ausgebildet, die im komprimierten Stentzustand neben reinen Schlitzbereichen flächig ausgeschnittene Bereiche einschließlich der Bereiche 35 umfassen.

Die Verbindungselemente 26 jeweils zweier in Längsrichtung 10 aufeinander folgender Verbindungsabschnitte 14 sind in Umfangsrichtung 24 so zueinander versetzt angeordnet, dass die Verbindungselemente 26 eines Verbindungsabschnitts 14 in Umfangsrichtung 24 betrachtet in der Mitte zwischen zwei Verbindungselementen 26 des anderen Verbindungsabschnitts 14 liegen. Konkret sind die Verbindungselemente 26 desselben Verbindungsabschnitts 14 jeweils um einen Umfangswinkel von 180° zueinander versetzt und zu den Verbindungselementen 26 des in Längsrichtung 10 benachbarten Verbindungsabschnitts 14 jeweils um einen Umfangswinkel von 90° versetzt. Zudem kehrt sich die Richtung des durch die Verbindungselemente 26 überbrückten Umfangsversatzes zwischen den durch die Verbindungselemente 26 jeweils verbundenen Bereichen 30 von einem Verbindungsabschnitt 14 zum nächsten um. Das heißt, während z.B. in Fig. 1 die Verbindungselemente 26 des am weitesten links angeordneten Verbindungsabschnitts 14 jeweils zwischen zwei Bereichen 30 von links unten nach rechts oben verlaufen, verlaufen die Verbindungselemente 26 des in Längsrichtung 10 darauf folgenden Verbindungsabschnitts 14 jeweils zwischen zwei Bereichen 30 von links oben nach rechts unten.

An den längsseitigen Enden des Stents sind jeweils mehrere Halteelemente 38 angeordnet, die einen Halsabschnitt 40 und einen demgegenüber verbreiterten Halteabschnitt 42 aufweisen. Diese Halteelemente 38 sind ebenfalls durch den röhrenförmigen Körper des Stents gebildet und dienen zur Festlegung des Stents an einem insbesondere ringförmigen Positionierelement eines Einführkatheters, welches beispielsweise Öffnungen aufweist, in die die Halteelemente 38 in dem an dem Einführkatheter festgelegten Zustand des Stents eingreifen, wobei die Halteelemente 38 mit dem Positionierelement in Längsrichtung 10 orientierte Hinterschneidungen zur Festlegung des Stents bilden können.

Fig. 2 zeigt einen Stent, welcher im Wesentlichen dem in Fig. 1 gezeigten Stent entspricht. Fig. 2 zeigt dabei eine seitliche Ansicht des röhrenförmigen expandierten Stents, wobei zur besseren Übersichtlichkeit nur die in Betrachtungsrichtung vorne gelegenen Bestandteile des Stents gezeigt sind und die Rückseite des Stents nicht dargestellt ist.

In dem in Fig. 2 dargestellten expandierten Zustand des Stents sind die Zellen 20 in Umfangsrichtung 24 zur Bildung von Rauten aufgeweitet. In Fig. 2 ist auch der in Umfangsrichtung 24 gerichtete, eine halbe Zellenbreite b betragende Versatz zwischen den Zellen 20 zweier aufeinander folgender Stützabschnitte 12 ersichtlich, welcher bei einer Verbiegung des Stents in Richtung senkrecht zur Längsrichtung 10 eine Kollision der freien Enden 21 der Zellen 20 verhindert.

Fig. 3 zeigt eine vergrößerte Darstellung einer Zelle 20 des in Fig. 1 oder 2 gezeigten Stents im expandierten Zustand einschließlich der Durchbrechung 16, der die Durchbrechung 16 umschließenden Umrandungselemente 18 und der abgerundeten freien Enden 21. Die Umrandungselemente 18 sind dabei im Wesentlichen gerade ausgebildet und schließen mit der Längsrichtung 10 des Stents einen Winkel α von etwa 30° ein. Durch diese Form wird eine sehr hohe radiale Aufstellkraft und Stützwirkung des Stents in den Stützabschnitten 12 erreicht.

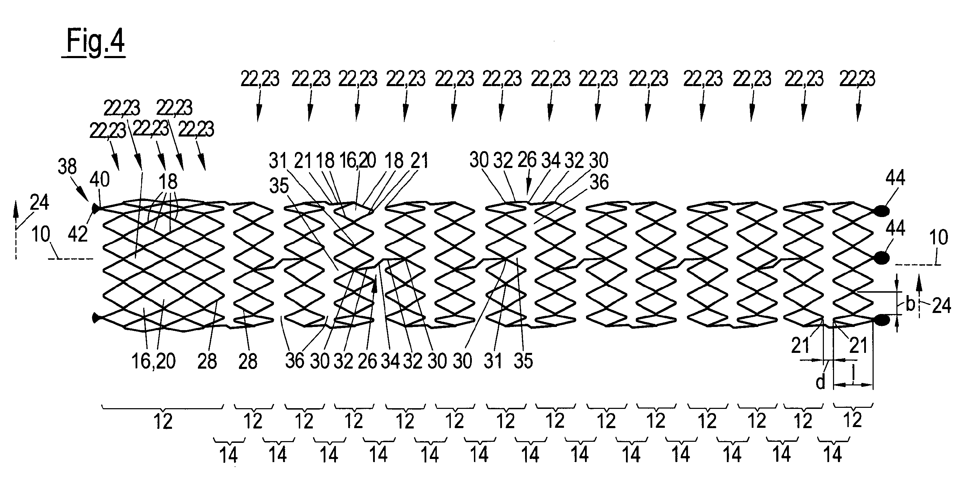

Fig. 4 zeigt einen Stent gemäß einer Ausführungsform der Erfindung, welcher im Wesentlichen dem in Fig. 2 gezeigten Stent entspricht. Der an dem in Fig. 4 links dargestellten längsseitigen Ende des Stents angeordnete Stützabschnitt 12 ist dabei allerdings nicht wie die übrigen Stützabschnitte 12 nur durch eine Reihe 22 von in Umfangsrichtung 24 aufeinander folgenden Zellen 20 gebildet, sondern durch insgesamt fünf in Längsrichtung 10 aufeinander folgende Reihen 22 von Zellen 20, die zusammen ein Rautengitternetz bilden, in dem sich die Zellen 20 sowohl in Längsrichtung 10 als auch in Umfangsrichtung 24 regelmäßig wiederholen. Im komprimierten Zustand bilden diese Durchbrechungen 16 sich in Längsrichtung 10 und Umfangsrichtung 24 regelmäßig wiederholende Schlitze.

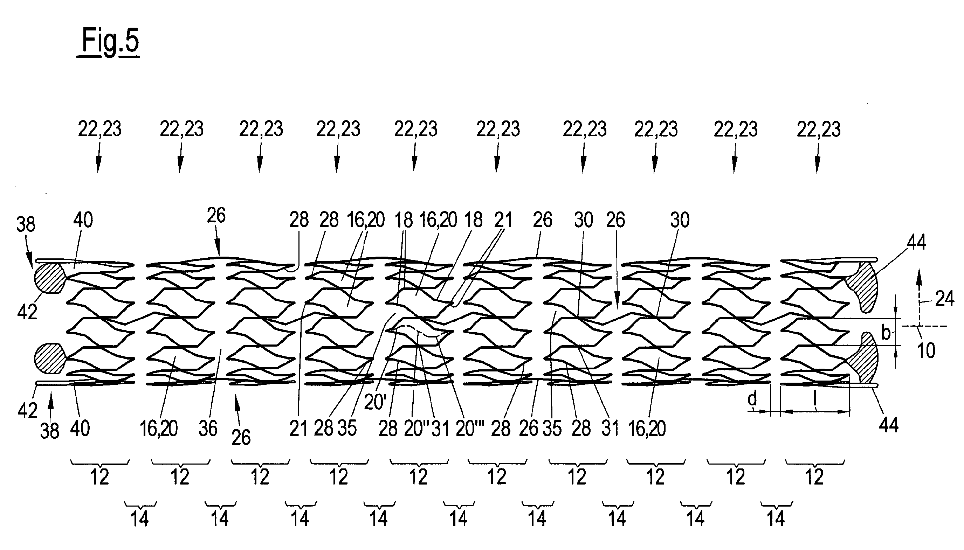

Fig. 5 zeigt einen Stent, welcher im Wesentlichen dem in Fig. 3 gezeigten Stent entspricht. Allerdings sind bei dieser Ausgestaltung die Zellen 20 der Stützabschnitte 12 im Wesentlichen S-oder wellenförmig ausgebildet und umfassen jeweils drei im expandierten und komprimierten Zustand schräg zur Längsachse 10 des Stents orientierte Abschnitte 20', 20", 20"'. Bei der in Fig. 5 gezeigten Ausführungsform sind die Zellen 20 von in Längsrichtung 10 aufeinander folgenden Stützabschnitten 12 nicht in Umfangsrichtung 24 zueinander versetzt angeordnet, so dass die freien Enden 21 der Zellen 20 der einander zugewandten Stirnseiten 28 einander direkt gegenüberstehen. Auch hier ist aber eine wie in Fig. 3 und 4 gezeigte Ausgestaltung möglich, bei der die Zellen 20 beispielsweise um eine halbe Zellenbreite b zueinander in Umfangsrichtung 24 versetzt angeordnet sind. Dementsprechend könnten die Z-förmigen Verbindungselemente 26 des in Fig. 5 gezeigten Stents in Bereichen 30 mit den Stirnseiten 28 der Stützabschnitte 12 verbunden sein, die zueinander in Umfangsrichtung 24 versetzt angeordnet sind, beispielsweise um eine halbe, eine oder eineinhalb Zellenbreiten b, anstatt wie in Fig. 5 gezeigt mit in Längsrichtung 10 auf einer Linie liegenden Bereichen 30 verbunden zu sein.

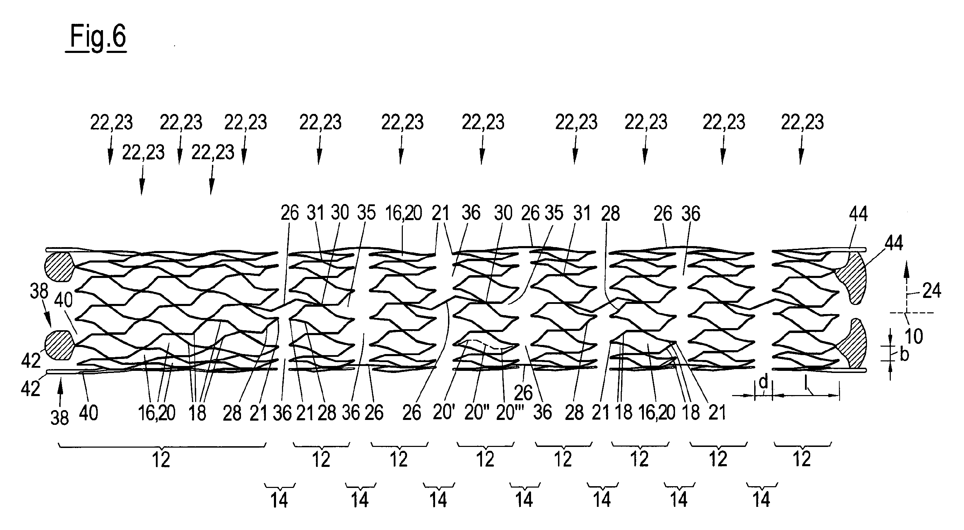

Fig. 6 zeigt einen Stent gemäß einer weiteren Ausführungsform der Erfindung, welcher im Wesentlichen dem in Fig. 5 gezeigten Stent entspricht. Dabei weist der in Fig. 6 am weitesten links gezeigte Stützabschnitt 12 des Stents allerdings fünf in Längsrichtung 10 aufeinander folgende Reihen 22 von in Umfangsrichtung 24 aufeinander folgenden Zellen 20 auf, die ein Gitternetz aus sich in Längsrichtung 10 und Umfangsrichtung 24 wiederholenden wellenförmigen Zellen 20 bilden. Dadurch wird die Steifigkeit des Stents in dem in Fig. 6 links dargestellten Endbereich erhöht.

### Bezugszeichenliste

- 10: Längsachse, Längsrichtung
- 12: Stützabschnitt
- 14: Verbindungsabschnitt
- 16: Durchbrechung
- 18: Umrandungselement
- 20: Zelle
- 20', 20", 20"': Abschnitt
- 21: freies Ende
- 22: Reihe
- 23: Ring
- 24: Umfangsrichtung
- 26: Verbindungselement
- 28: Stirnseite
- 30, 31: Verbindungsbereich
- 32, 34: Abschnitt eines Verbindungselements
- 35: flächig ausgeschnittener Bereich
- 36: Öffnung
- 38: Halteelement
- 40: Halsabschnitt
- 42: Halteabschnitt
- 44: Markierelement
- α: Winkel
- b: Zellenbreite
- d: Abstand
- 1: Zellenlänge

## Patentansprüche

1. Stent zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der von einem komprimierten Zustand mit einem ersten Querschnittsdurchmesser in einen expandierten Zustand mit einem vergrößerten zweiten Querschnittsdurchmesser überführbar ist,
wobei der Stent wenigstens zwei Stützabschnitte (12) und zumindest einen Verbindungsabschnitt (14) umfasst, die in Längsrichtung (10) des Stents aufeinander folgen,
wobei die Stützabschnitte (12) jeweils mehrere Durchbrechungen (16) der Wand des röhrenförmigen Körpers und durch den röhrenförmigen Körper gebildete Umrandungselemente (18) aufweisen, die die Durchbrechungen (16) umschließen und zusammen mit diesen im expandierten Zustand Zellen (20) der Stützabschnitte (12) bilden, wobei zwei in Längsrichtung (10) benachbarte Stützabschnitte (12) über einen dazwischen liegenden Verbindungsabschnitt (14) miteinander verbunden sind,
wobei einander zugewandte Stirnseiten (28) der benachbarten Stützabschnitte (12) jeweils durch eine Reihe (22) von stirnseitigen Zellen (20) des jeweiligen Stützabschnitts (12) gebildet sind,
wobei der Verbindungsabschnitt (14) ein oder mehrere durch den röhrenförmigen Körper gebildete Verbindungselemente (26) umfasst, die die einander zugewandten Stirnseiten (28) der zwei benachbarten Stützabschnitte (12) miteinander verbinden,
und wobei nur ein Teil der Zellen (20), die eine der zwei einander zugewandten Stirnseiten (28) der benachbarten Stützabschnitte (12) bilden, über ein Verbindungselement (26) direkt mit der anderen der zwei einander zugewandten Stirnseiten (28) der benachbarten Stützabschnitte (12) verbunden ist
**dadurch gekennzeichnet,**
**dass** der Stent einen an seinem längsseitigen Ende angeordneten endseitigen Stützabschnitt (12) sowie einen sich an diesen endseitigen Stützabschnitt (12) anschließenden mittleren Bereich des Stents umfasst,
wobei der mittlere Bereich des Stents durch eine Vielzahl von in Längsrichtung benachbarten, durch Verbindungsabschnitte (14) miteinander verbundenen Stützabschnitten (12) gebildet ist, die jeweils genau eine Reihe (22) von Zellen (20) aufweisen, die in Umfangsrichtung (24) des Stents aufeinander folgen, und
wobei der endseitige Stützabschnitt (12) mehrere in Längsrichtung (10) des Stents aufeinander folgende Reihen (22) von Zellen (20) aufweist, wobei die Zellen (20) einer Reihe (22) jeweils in Umfangsrichtung (24) des Stents aufeinander folgend angeordnet sind, so dass der endseitige Stützabschnitt (12) eine größere axiale Länge und eine größere Anzahl von in Längsrichtung (10) des Stents aufeinander folgenden Reihen (22) von in Umfangsrichtung (24) des Stents aufeinander folgenden Zellen (20) aufweist als die im mittleren Bereich des Stents angeordneten Stützabschnitte (12).

2. Stent nach Anspruch 1,
**dadurch gekennzeichnet, dass** die Anzahl der Verbindungselemente (26) des Verbindungsabschnitts (14) geringer ist als die Anzahl derjenigen Zellen (20) zumindest eines und insbesondere beider der benachbarten Stützabschnitte (12), die eine der zwei über die Verbindungselemente (26) des Verbindungsabschnitts (14) miteinander verbundenen und einander zugewandten Stirnseiten (28) bilden.

3. Stent nach Anspruch 1 oder 2,
**dadurch gekennzeichnet, dass** zumindest einer und insbesondere beide benachbarten Stützabschnitte (12) jeweils eine Reihe (22) von Zellen (20) umfasst, die in Umfangsrichtung (24) des Stents aufeinander folgen und bevorzugt einen geschlossenen, in Umfangsrichtung (24) des Stents umlaufenden Ring (23) bilden.

4. Stent nach zumindest einem der vorstehenden Ansprüche,
**dadurch gekennzeichnet, dass** die die Stirnseite (28) eines Stützabschnitts (12) bildenden Zellen (20) jeweils ein freies Ende (21) aufwiesen, das dem jeweils benachbarten Stützabschnitt (12) zugewandt ist und welches eine abgerundete Form aufweist.

5. Stent nach zumindest einem der vorstehenden Ansprüche,
**dadurch gekennzeichnet, dass** die einander zugewandten stirnseitigen Zellen (20) zweier benachbarter Stützabschnitte (12) in Umfangsrichtung (24) des Stents zueinander versetzt angeordnet sind.

6. Stent nach zumindest einem der vorstehenden Ansprüche,
**dadurch gekennzeichnet, dass** die Zellen (20) der Stützabschnitte (12) im expandierten Zustand des Stents im Wesentlichen rautenförmig ausgebildet sind und/oder zumindest einen und vorzugsweise drei schräg zu der Längsachse des Stents angeordnete Abschnitte (20', 20", 20"') aufweisen.

7. Stent nach zumindest einem der vorstehenden Ansprüche,
**dadurch gekennzeichnet, dass** zumindest ein Verbindungselement (26) des Verbindungsabschnitts (14) im expandierten und/oder komprimierten Zustand des Stents eine in Längsrichtung (10) des Stents gemessene Länge aufweist, die zumindest 50 %, zumindest 75 %, zumindest 100 % oder mehr der Länge (l) der durch das Verbindungselement (26) verbundenen Zellen (20) beträgt.

8. Stent nach zumindest einem der vorstehenden Ansprüche,
**dadurch gekennzeichnet, dass** zumindest ein Verbindungselement (26) des Verbindungsabschnitts (14) in einem Bereich (30) mit der Stirnseite (28) eines Stützabschnitts (12) verbunden ist, in dem zwei die Stirnseite (28) bildende Zellen (20) dieses Stützabschnitts (12) miteinander verbunden sind.

9. Stent nach zumindest einem der vorstehenden Ansprüche,
**dadurch gekennzeichnet, dass** zumindest ein Verbindungselement (26) des Verbindungsabschnitts (14) zumindest abschnittsweise quer zur Längsrichtung (10) des Stents orientiert ist.

10. Stent nach zumindest einem der vorstehenden Ansprüche,
**dadurch gekennzeichnet, dass** das wenigstens eine Verbindungselement (26) des Verbindungsabschnitts (14) eine geknickte oder gebogene Form aufweist und vorzugsweise im Wesentlichen S- oder Z-förmig ausgebildet ist.

11. Stent nach zumindest einem der vorstehenden Ansprüche,
**dadurch gekennzeichnet, dass** der Stent mehr als zwei Stützabschnitte (12) und mehrere Verbindungsabschnitte (14) aufweist, die in Längsrichtung (10) des Stents aufeinander folgen, wobei ein Verbindungsabschnitt (14) jeweils zwei zueinander benachbarte Stützabschnitte (12) miteinander verbindet.

12. Stent nach Anspruch 11,
**dadurch gekennzeichnet, dass** die Verbindungselemente (26) zweier, insbesondere zueinander benachbarter, Verbindungsabschnitte (14) in Umfangsrichtung (24) des Stents zueinander versetzt angeordnet sind.

13. Stent nach zumindest einem der vorstehenden Ansprüche,
**dadurch gekennzeichnet, dass** der wenigstens eine Verbindungsabschnitt (14) zwischen einem und zehn Verbindungselementen und bevorzugt ein, zwei, drei oder vier Verbindungselemente (26) umfasst.

14. Stent nach zumindest einem der vorstehenden Ansprüche,
**dadurch gekennzeichnet, dass** der wenigstens eine Verbindungsabschnitt (14) mehrere Verbindungselemente (26) umfasst, die in zumindest näherungsweise gleichen Winkelabständen über den Umfang des Stents verteilt angeordnet sind.

15. Stent nach zumindest einem der vorstehenden Ansprüche,
**dadurch gekennzeichnet, dass** zumindest eine Stirnseite (28) eines Stützabschnitts (12) durch zwischen zwei und vierzig, vorzugsweise zwischen vier und dreißig und besonders bevorzugt zwischen acht und vierundzwanzig Zellen (20) gebildet ist.

## Claims

1. A stent for transluminal implantation into hollow organs, in particular into blood vessels, ureters, esophagi, the colon, the duodenum or the biliary tract, having a substantially tubular body which can be converted from a compressed state having a first cross-sectional diameter into an expanded state having an enlarged second cross-sectional diameter,
wherein the stent comprises at least two support sections (12) and at least one connection section (14) which follow one another in the longitudinal direction (10) of the stent;
wherein the support sections (12) each have a plurality of apertures (16) of the wall of the tubular body and bordering elements (18) which are formed by the tubular body, which surround the apertures (16) and which form cells (20) of the support sections (12) together with them in the expanded state;
wherein two support sections (12) adjacent in the longitudinal direction (10) are connected to one another via a connection section (14) disposed therebetween;
wherein mutually facing end faces (28) of the adjacent support sections (12) are each formed by a row (22) of end-face cells (20) of the respective support section (12);
wherein the connection section (14) comprises one or more connection elements (26) which are formed by the tubular body and which connect the mutually facing end faces (28) of the two adjacent support sections (12) to one another,
and wherein only some of the cells (20), which form one of the two mutually facing end faces (28) of the adjacent support sections (12), are directly connected to the other one of the two mutually facing end faces (28) of the adjacent support sections (12) via a connection element (26),
**characterized in that** the stent comprises an end-side support section (12) arranged at its end at the long side as well as a central region of the stent adjoining this end-side support section (12), with the central region of the stent being formed by a plurality of support sections (12) which are adjacent in the longitudinal direction, which are connected to one another by connection sections (14) and which each have exactly one row (22) of cells (20) which follow one another in the peripheral direction (24) of the stent; and
with the end-side support section (12) having a plurality of rows (22) of cells (20) which follow one another in the longitudinal direction (10) of the stent, with the cells (20) of one row (22) each being arranged following one another in the peripheral direction (24) of the stent,
such that the end-side support section (12) has a larger axial length and a larger number of rows (22) of cells (20), the rows following one another in the longitudinal direction (10) of the stent and the cells following one another in the peripheral direction (24) of the stent, than the support sections (12) arranged in the central region of the stent.

2. A stent in accordance with claim 1,
**characterized in that**
the number of connection elements (26) of the connection section (14) is smaller than the number of those cells (20) of at least one of the adjacent support sections, and in particular of both of the adjacent support sections (12), which form one of the two mutually facing end faces (28) connected to one another via the connection elements (26) of the connection section (14).

3. A stent in accordance with claim 1 or claim 2,
**characterized in that**
at least one adjacent support section, and in particular both adjacent support sections (12), respectively comprise(s) a row (22) of cells (20) which follow one another in the peripheral direction (24) of the stent and which preferably form a closed ring (23) running around in the peripheral direction (24) of the stent.

4. A stent in accordance with at least one of the preceding claims,
**characterized in that**
the cells (20) forming the end face (28) of a support section (12) each have a free end (21) which faces the respective adjacent support section (12) and which has a rounded shape.

5. A stent in accordance with at least one of the preceding claims,
**characterized in that**
the mutually facing end-face cells (20) of two adjacent support sections (12) are arranged offset from one another in the peripheral direction (24) of the stent.

6. A stent in accordance with at least one of the preceding claims,
**characterized in that**
the cells (20) of the support sections (12) are substantially diamond-shaped in the expanded state of the stent and/or have at least one section and preferably three sections (20', 20", 21'") arranged obliquely to the longitudinal axis of the stent.

7. A stent in accordance with at least one of the preceding claims,
**characterized in that**
at least one connection element (26) of the connection section (14) has a length measured in the longitudinal direction (10) of the stent in the expanded and/or compressed state of the stent which amounts to at least 50%, at least 75%, at least 100% or more of the length (l) of the cells (20) connected by the connection element (26).

8. A stent in accordance with at least one of the preceding claims,
**characterized in that**
at least one connection element (26) of the connection section (14) is connected to the end face (28) of a support section (12) in a region (30) in which two cells (20) of this support section (12) forming the end face (28) are connected to one another.

9. A stent in accordance with at least one of the preceding claims,
**characterized in that**
at least one connection element (26) of the connection section (14) is oriented at least sectionally transverse to the longitudinal direction (10) of the stent.

10. A stent in accordance with at least one of the preceding claims,
**characterized in that**
the at least one connection element (26) of the connection section (14) has a kinked or curved shape and is preferably substantially of S shape or of Z shape.

11. A stent in accordance with at least one of the preceding claims,
**characterized in that**
the stent has more than two support sections (12) and a plurality of connection sections (14) which follow one another in the longitudinal direction (10) of the stent, with a connection section (14) respectively connecting two mutually adjacent support sections (12) to one another.

12. A stent in accordance with claim 11,
**characterized in that**
the connection elements (26) of two connection sections (14), in particular adjacent to one another, are arranged offset from one another in the peripheral direction (24) of the stent.

13. A stent in accordance with at least one of the preceding claims,
**characterized in that**
the at least one connection section (14) comprises between one and ten connection elements and preferably one, two, three or four connection elements (26).

14. A stent in accordance with at least one of the preceding claims,
**characterized in that**
the at least one connection section (14) comprises a plurality of connection elements (26) which are arranged at at least approximately the same angular intervals distributed over the periphery of the stent.

15. A stent in accordance with at least one of the preceding claims,
**characterized in that**
at least one end face (28) of a support section (12) is formed by between two and forty, preferably between four and thirty, and particularly preferably between eight and twenty-four cells (20).

## Revendications

1. Stent destiné à l'implantation transluminale dans des organes creux, en particulier dans des vaisseaux sanguins, des voies urinaires, des oesophages, des colons, des duodénums ou des voies biliaires, comportant un corps sensiblement en forme tubulaire qui est transformable d'un état comprimé présentant un premier diamètre de section transversale en un état expansé présentant un second diamètre de section transversale agrandi,
dans lequel
le stent comprend au moins deux portions d'appui (12) et au moins une portion de liaison (14) qui se succèdent en direction longitudinale (10) du stent,
les portions d'appui (12) présentent chacune plusieurs traversées (16) de la paroi du corps tubulaire ainsi que des éléments d'entourage (18) constitués par les corps tubulaires qui entourent les traversées (16) et qui, dans l'état expansé, constituent conjointement avec celles-ci des cellules (20) des portions d'appui (12), deux portions d'appui (12) voisines en direction longitudinale (10) étant reliées l'une à l'autre via une portion de liaison (14) située entre les deux,
des faces frontales (28) tournées l'une vers l'autre des portions d'appui (12) voisines sont formées par une rangée respective (22) de cellules frontales (20) de la portion d'appui respective (12),
la portion de liaison (14) comprend un ou plusieurs éléments de liaison (26) formés par le corps tubulaire, qui relient les faces frontales (28) tournées l'une vers l'autre des deux portions d'appui (12) voisines,
une partie seulement des cellules (20) formant l'une des deux faces frontales (28) tournées l'une vers l'autre des portions d'appui (12) voisines est reliée directement à l'autre des deux faces frontales (28) tournées l'une vers l'autre des portions d'appui (12) voisines via un élément de liaison (26),
**caractérisé en ce que**
le stent comprend une partie d'appui (12) terminale agencée à son extrémité longitudinale, ainsi qu'une zone médiane qui se raccorde à cette portion d'appui terminale (12),
la zone médiane du stent est formée par une multitude de portions d'appui (12) voisines en direction longitudinale et reliées les unes aux autres par des portions de liaison (14) qui présentent chacune précisément une rangée (22) de cellules (20) qui se succèdent en direction périphérique (24) du stent, et
la portion d'appui (12) terminale présente plusieurs rangées (22) de cellules (20) qui se succèdent en direction longitudinale (10) du stent, les cellules (20) d'une rangée (22) étant agencées de manière à se succéder chacune en direction périphérique (24) du stent, de sorte que la portion d'appui (12) terminale présente une longueur axiale et un nombre de rangées (22), se succédant en direction longitudinale (10) du stent, de cellules (20) se succédant en direction périphérique (24) du stent, longueur et nombre qui sont plus élevés que ceux des portions d'appui (12) agencées dans la zone médiane du stent.

2. Stent selon la revendication 1,
**caractérisé en ce que**
le nombre d'éléments de liaison (26) de la portion de liaison (14) est inférieur au nombre de celles des cellules (20) d'au moins une et en particulier des deux portions d'appui (12) voisines, qui constituent l'une des deux faces frontales (28) tournées l'une vers l'autre et reliées l'une à l'autre par les éléments de liaison (26) de la portion de liaison (14).

3. Stent selon la revendication 1 ou 2,
**caractérisé en ce que**
l'une au moins et en particulier les deux portions d'appui voisines (12) comprend/comprennent chacune une range (22) de cellules (20) qui se succèdent en direction périphérique (24) du stent et qui forment de préférence un anneau fermé (23) s'étendant en direction périphérique (24) du stent.

4. Stent selon l'une au moins des revendications précédentes,
**caractérisé en ce que**
les cellules (20) formant la face frontale (28) d'une portion d'appui (12) présentent chacune une extrémité libre (21) qui est tournée vers la portion d'appui voisine respective (12) et qui présente une forme arrondie.

5. Stent selon l'une au moins des revendications précédentes,
**caractérisé en ce que**
les cellules frontales (20) tournées l'une vers l'autre de deux portions d'appui voisines (12) sont agencées en étant décalées l'une par rapport à l'autre en direction périphérique (24) du stent.

6. Stent selon l'une au moins des revendications précédentes,
**caractérisé en ce que**
dans l'état expansé du stent les cellules (20) des portions d'appui (12) sont de forme sensiblement en losange et/ou présentent au moins une et de préférence trois portions (20', 20", 20"') agencées en oblique par rapport à l'axe longitudinal du stent.

7. Stent selon l'une au moins des revendications précédentes,
**caractérisé en ce que**
dans l'état expansé et/ou comprimé du stent, au moins un élément de liaison (26) de la portion de liaison (14) présente une longueur mesurée en direction longitudinale (10) du stent qui s'élève à au moins 50 %, au moins 75 %, au moins 100 % ou plus de la longueur (1) des cellules (20) reliées par l'élément de liaison (26).

8. Stent selon l'une au moins des revendications,
**caractérisé en ce que**
au moins un élément de liaison (26) de la portion de liaison (14) est relié à la face frontale (28) d'une portion d'appui (12) dans une zone (30) dans laquelle deux cellules (20) de cette portion d'appui (12) formant la face frontale (28) sont reliées l'une à l'autre.

9. Stent selon l'une au moins des revendications précédentes,
**caractérisé en ce que**
au moins un élément de liaison (26) de la portion de liaison (14) est au moins localement orienté transversalement à la direction longitudinale (10) du stent.

10. Stent selon l'une au moins des revendications précédentes,
caractérisé en que
au moins un élément de liaison (26) de la portion de liaison (14) présente une forme pliée ou recourbée et est réalisé de préférence sensiblement en forme de S ou de Z.

11. Stent selon l'une au moins des revendications précédentes,
**caractérisé en ce que**
le stent comprend plus de deux portions d'appui (12) et plusieurs portions de liaison (14) qui se succèdent en direction longitudinale (10) du stent, une portion de liaison (14) reliant l'une à l'autre deux portions d'appui (12) voisines respectives.

12. Stent selon la revendication 11,
**caractérisé en ce que**
les éléments de liaison (26) de deux portions de liaison (14) en particulier voisines sont agencés en décalage l'un de l'autre en direction périphérique (14) du stent.

13. Stent selon l'une au moins des revendications précédentes,
**caractérisé en ce que**
ladite au moins une portion de liaison (14) comprend entre un et dix éléments de liaison et de préférence un, deux, trois ou quatre éléments de liaison (26).

14. Stent selon l'une au moins des revendications précédentes,
**caractérisé en ce que**
ladite au moins une portion de liaison (14) comprend plusieurs éléments de liaison (26) qui sont agencés en étant répartis sur la périphérie du stent à des distances angulaires au moins approximativement égales.

15. Stent selon l'une au moins des revendications précédentes,
**caractérisé en ce que**
au moins une face frontale (28) d'une portion d'appui (12) est formée par un nombre de cellules (20) compris entre deux et quarante, de préférence entre quatre et trente et de manière particulièrement préférée entre huit et vingt-quatre.
